# EUROPÄISCHE PATENTSCHRIFT

(11) **EP 0 832 072 B1**
(45) Veröffentlichungstag und Bekanntmachung des Hinweises auf die Patenterteilung: **24.09.2003**
(21) Anmeldenummer: 96917423.4
(22) Anmeldetag: 24.05.1996
(51) Int. Cl.: C07D 237/14, C07D 237/22, C07D 237/16, A01N 43/58

(54) **PHENYLPYRIDAZINONE**
PHENYLPYRIDAZINONES
PHENYLPYRIDAZINONES

(30) Priorität: 06.06.1995 DE 19520613
(43) Veröffentlichungstag der Anmeldung: 01.04.1998
(73) Patentinhaber: Bayer CropScience AG, 40789 Monheim (DE)
(72) Erfinder: LINKER, Karl-Heinz, D-51377 Leverkusen (DE); FINDEISEN, Kurt, D-51375 Leverkusen (DE); HAAS, Wilhelm, D-50259 Pulheim (DE); DOLLINGER, Markus, D-51381 Leverkusen (DE); SANTEL, Hans-Joachim, D-51371 Leverkusen (DE)
(86) Internationale Anmeldenummer: EP9602246
(87) Internationale Veröffentlichungsnummer: WO96039392

(56) Entgegenhaltungen:
- EP-A- 0 003 805
- EP-A- 0 210 647
- EP-A- 0 224 094
- CH-A- 373 919
- FR-A- 2 381 033

## Beschreibung

Die Erfindung betrifft neue Phenylpyridazinone, Verfahren zu ihrer Herstellung, ihre Verwendung als Herbizide sowie neue Zwischenprodukte und deren Herstellungsverfahren.

Es ist bekannt, dass bestimmte substituierte Phenylpyridazinone, wie z.B. die Verbindungen 5-Amino-4-chlor-2-phenyl-pyridazin-3-on und 4-Brom-5-methyl-2-phenylpyridazin-3-on herbizide Eigenschaften auf weisen (vgl. DE 1105232 und DE 2706700; vgl auch DE 1670309, DE 1670315, DE 1695840, DE 2526643, DE 2808193, DE 2821809 und US 5298502). Die herbizide Wirksamkeit dieser Verbindungen ist jedoch nicht in allen Belangen zufriedenstellend.

Es wurden nun die neuen substituierten Phenylpyridazinone der allgemeinen Formel (I) gefunden, 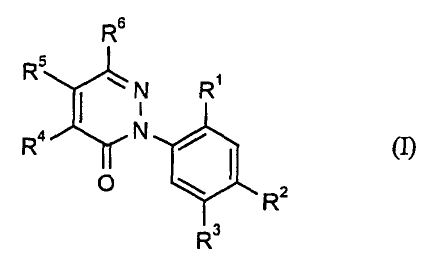 in welcher
- R¹: für Fluor, Chlor oder Brom steht,
- R²: für Cyano, Carbamoyl, Thiocarbamoyl, Fluor, Chlor, Brom, oder für jeweils gegebenenfalls durch Fluor und/oder Chlor substituiertes Alkyl, Alkoxy oder Alkylthio mit jeweils 1 oder 2 Kohlenstoffatomen steht,
- R³: für die Gruppierung -A¹-A²-A³ steht,
in welcher
A¹ für eine Einfachbindung, für Sauerstoff oder die Gruppierung -N-A⁴-steht, worin A⁴ für Wasserstoff, Hydroxy, C₁-C₄-Alkyl, C₃-C₄-Alkenyl, C₃-C₄-Alkinyl, C₁-C₄-Alkoxy, Phenyl, C₁-C₄-Alkylsulfonyl oder Phenylsulfonyl steht,
A² für eine Einfachbindung oder für gegebenenfalls durch Fluor, Chlor oder Brom substituiertes C₁-C₆-Alkandiyl steht,
A³ für Fluor, Chlor, Brom steht,
A³ weiterhin für jeweils gegebenenfalls durch Fluor, Chlor oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy-(thio)phosphoryl mit jeweils 1 bis 6 Kohlenstoffatomen in den Alkyl-gruppen steht,
A³ weiterhin für jeweils gegebenenfalls durch Fluor oder Chlor substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen steht, und
- R⁴, R⁵ und R⁶: gleich oder verschieden sind und für Wasserstoff, Cyano, Thiocarbamoyl, Nitro, Hydroxy, Mercapto, Amino, Fluor, Chlor, Brom, oder für jeweils gegebenenfalls durch Fluor und/oder Chlor substituiertes Alkyl, Alkoxy, Alkylthio, Alkylamino oder Dialkylamino mit jeweils 1 bis 4 Kohlenstoffatomen in den Alkylgruppen stehen,
wobei die vorbekannten Verbindungen 2-(2,4,5-Trichlor-phenyl)-pyridazin-3-on (vgl. Liebigs Ann. Chem. 697 (1966), 42-61) und 4-Chlor-5-dimethylamino-2-(4-chlor-2-fluor-5-propargyloxy-phenyl)-pyridazin-3-on (vgl. US 5298502) durch Disclaimer ausgenommen sind.

Die Erfindung betrifft insbesondere Verbindungen der Formel (I), in welcher
- R¹: für Fluor oder Chlor steht,
- R²: für Cyano, Thiocarbamoyl, Fluor, Chlor, Brom, Methyl oder Trifluormethyl steht,
- R³: für die Gruppierung -A¹-A²-A³ steht,
in welcher
A¹ für eine Einfachbindung, für Sauerstoff oder die Gruppierung -N-A⁴-steht, worin A⁴ für Wasserstoff, Hydroxy, Methyl, Ethyl, n- oder i-Propyl, Methoxy, Ethoxy, n- oder i-Propoxy, Methylsulfonyl oder Ethylsulfonyl steht,
A² für eine Einfachbindung oder für Methylen, Ethan-1,1-diyl, Ethan-1,2-diyl, Fropan-1,1-diyl, Propan-1,2-diyl oder Propan-1,3-diyl steht,
A³ für Fluor, Chlor, Brom steht,
A³ weiterhin für jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, n-, i-, s- oder t-Pentyloxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Methoxycarbonyl, Ethoxycarbonyl, n- oder i-Propoxycarbonyl, Dimethoxyphosphoryl, Diethoxyphosphoryl oder Dipropoxyphosphoryl, Diisopropoxyphosphoryl steht,
A³ weiterhin für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl steht, und
R⁴, R⁵ und R⁶ gleich oder verschieden sind und für Wasserstoff, Cyano, Thiocarbamoyl, Nitro, Hydroxy, Mercapto, Amino, Fluor, Chlor, Brom, oder für jeweils gegebenenfalls durch Fluor und/oder Chlor substituiertes Methyl, Ethyl, n- oder i-Propyl, n-, i-, s- oder t-Butyl, Methoxy, Ethoxy, Methylthio, Ethylthio, Methylamino, Ethylamino oder Dimethylamino stehen,
wobei die vorbekannten Verbindungen 2-(2,4,5-Trichlor-phenyl)-pyridazin-3-on (vgl. Liebigs Ann. Chem. 697 (1966), 42-61) und 4-Chlor-5-dimethylamino-2-(4-chlor-2-fluor-5-propargyloxy-phenyl)-pyridazin-3-on (vgl. US 5298502) durch Disclaimer ausgenommen sind.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen bevorzugten Bereichen beliebig kombiniert werden.

Man erhält die neuen substituierten Phenylpyridazinone der allgemeinen Formel (I), wenn man
(a) Halogenarene der allgemeinen Formel (II) 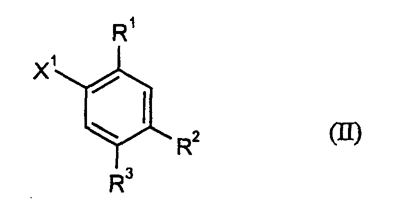 in welcher
   - R¹, R² und R³: die oben angegebenen Bedeutungen haben und
   - X¹: für Halogen steht,
   mit Pyridazinonen der allgemeinen Formel (III) 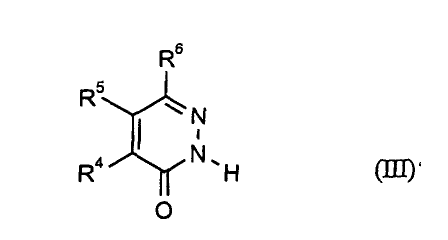 in welcher
   R⁴, R⁵ und R⁶ die oben angegebenen Bedeutungen haben,
   - oder mit Säureaddukten oder Alkalimetallsalzen von Verbindungen der Formel (III) -
   gegebenenfalls in Gegenwart eines Reaktionshufsmittels und gegebenenfalls in Gegenwart eines Verdünnungsmittels umsetzt,
   oder wenn man
(b) Arylhydrazine der allgemeinen Formel (IV) 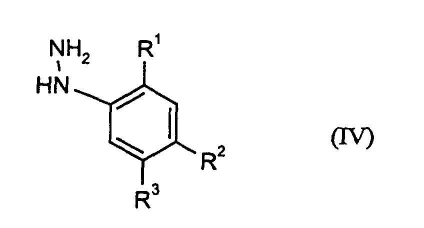 in welcher
   R¹, R² und R³ die oben angegebenen Bedeutungen haben,
   mit β-Trihalomethyl-enonen der allgemeinen Formel (V) 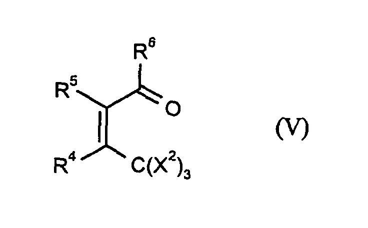 in welcher
   - R⁴, R⁵ und R⁶: die oben angegebenen Bedeutungen haben und
   - X²: für Halogen steht,
   gegebenenfalls in Gegenwart eines Reaktionshilfsmittels und gegebenenfalls in Gegenwart eines Verdünnungsmittels umsetzt,
   oder wenn man
(c) Hydrazoncarbonsäuren der allgemeinen Formel (VI) 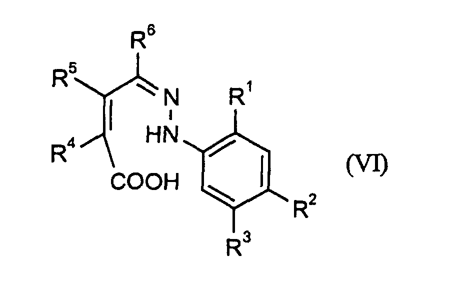 in welcher
   R¹, R², R³, R⁴, R⁵ und R⁶ die oben angegebenen Bedeutungen haben, cyclisierend kondensiert, d.h. mit einem wasser-entziehenden Mittel umsetzt,
   oder wenn man
(d) 2,4-disubstituierte Phenylpyridazinone der allgemeinen Formel (Ia) 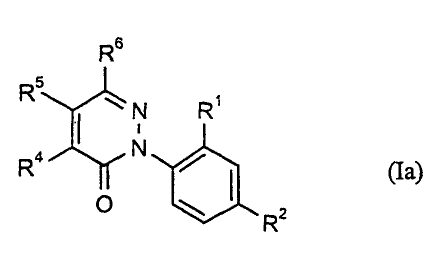
in welcher
R¹, R², R⁴, R⁵ und R⁶ die oben angegebenen Bedeutungen haben,
nitriert, d.h. mit einem Nitrierungsmittel umsetzt.

Die Verbindungen der allgemeinen Formel (I) können auch nach weiteren üblichen Methoden in andere Verbindungen der allgemeinen Formel (I) gemäß obiger Definition umgewandelt werden, beispielsweise durch nucleophile Substitution (z.B. R³: F → OH, SH, NH₂, OCH3, NHSO₂CH₃; R5: Cl → N(CH₃)₂) oder durch weitere Umwandlungen funktioneller Gruppen (z.B. R²: CONH₂ → CN, CN → CSNH₂; R³: NO₂ → NH₂, NH₂ → F, Cl, Br, CN, NHSO₂CH₃, SO₂Cl) - vgl. auch die Herstellungsbeispiele.

Die neuen substituierten Phenylpyridazinone der allgemeinen Formel (I) zeichnen sich durch starke herbizide Wirksamkeit aus.

In den Definitionen sind die gesättigten oder ungesättigten Kohlenwasserstoffketten, wie Alkyl, Alkenyl oder Alkinyl jeweils geradkettig oder verzweigt.

Halogen steht im Allgemeinen für Fluor, Chlor, Brom oder Iod, vorzugsweise für Fluor, Chlor oder Brom, insbesondere für Fluor oder Chlor.

Beispiele für die erfindungsgemäßen Verbindungen der Formel (I) sind in den nachstehenden Gruppen aufgeführt.

### Gruppe 1

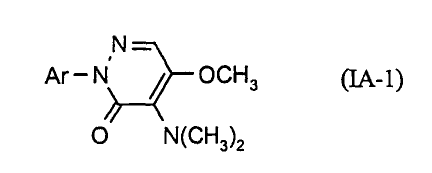

Ar hat hierbei beispielhaft die im Folgenden aufgeführten Bedeutungen:
2,4,5-Trichlor-phenyl, 2,4-Dichlor-5-fluor-phenyl, 2-Chlor-4,5-difluor-phenyl, 4-Chlor-2,5-difluor-phenyl, 5-Chlor-2,4-difluor-phenyl, 2-Fluor-5-chlor-4-cyano-phenyl, 2,4,5-Trifluor-phenyl, 2,5-Dichlor-4-cyano-phenyl, 2-Chlor-5-fluor-4-cyano-phenyl, 2,5-Difluor-4-cyano-phenyl, 2-Chlor-4-cyano-5-methyl-phenyl, 2,4-Dichlor-5-methoxy-phenyl, 2,4-Dichlor-5-ethoxy-phenyl, 2,4-Dichlor-5-n-propoxy-phenyl, 2,4-Dichlor-5-i-propoxy-phenyl, 4-Chlor-2-fluor-5-methoxy-phenyl, 4-Chlor-2-fluor-5-ethoxy-phenyl, 4-Chlor-2-fluor-5-n-propoxy-phenyl, 4-Chlor-2-fluor-5-i-propoxyphenyl, 2-Fluor-4-cyano-5-methyl-phenyl, 2,4-Dichlor-5-methyl-phenyl, 2-Chlor-4-cyano-5-trifluormethyl-phenyl, 2-Fluor-4-cyano-5-trifluormethyl-phenyl, 2-Chlor-4-methyl-5-trifluormethyl-phenyl, 2-Chlor-5-fluor-4-methoxy-phenyl, 2-Fluor-4-methoxy-5-methyl-phenyl, 2,5-Difluor-4-thiocarbamoyl-phenyl, 2-Chlor-4-fluor-5-ipropoxy-phenyl, 2-Fluor-4-cyano-5-methoxy-phenyl, 2-Fluor-4-cyano-5-i-propoxyphenyl, 2-Chlor-4-cyano-5-(2-propinyloxy)-phenyl, 2-Fluor-4-cyano-5-(1-methyl-2-propinyloxy)-phenyl, 2-Chlor-4-thiocarbamoyl-5-i-propoxy-phenyl, 2-Fluor-4-cyano-5-(2-propenyloxy)-phenyl, 2-Fluor-4-chlor-5-(2-propenyloxy)-phenyl, 2-Chlor-4-cyano-5-methylsulfonylamino-phenyl, 2-Fluor-4-cyano-5-ethylsulfonylamino-phenyl, 2-Fluor-4-thiocarbamoyl-5-methylsulfonyl-phenyl, 2-Chlor-4-cyano-5-ethylsulfonylamino-phenyl, 2-Fluor-4-cyano-5-i-propylsulfonylamino-phenyl, 2-Chlor-4-thiocarbamoyl-5-ethylsulfonylamino-phenyl, 2-Fluor-4-cyano-5-trifluormethylsulfonylamino-phenyl, 2-Fluor-4-cyano-5-(2,2-difluorethylsulfonylamino)-phenyl, 2-Fluor-4-cyano-5-phenylsulfonylamino-phenyl, 2-Fluor-4-cyano-5-t-butylsulfonylamino-phenyl, 2-Chlor-4-cyano-5-methoxycarbonyl-phenyl, 2-Fluor-4-cyano-5-ethoxycarbonylphenyl, 2-Fluor-4-thiocarbamoyl-5-methoxycarbonyl-phenyl, 2-Fluor-4-cyano-5-methylamino-phenyl, 2-Chlor-4-cyano-5-(N-methyl-ethylsulfonylamino)-phenyl, 2-Chlor-4-cyano-5-i-propoxycarbonyl-phenyl, 2-Fluor-4-cyano-5-(bis-ethylsulfonylamino)-phenyl, 2-Fluor-4-cyano-5-(N-methylsulfonyl-ethylsulfonylamino)-phenyl, 2-Chlor-4-cyano-5-dimethylamino-phenyl, 2-Fluor-4-cyano-5-methylsulfonyloxyphenyl, 2-Chlor-4-cyano-5-difluormethoxy-phenyl, 2-Fluor-4-cyano-5-(2-chlor-2-propenyloxy)-phenyl, 2-Fluor-4-cyano-5-diethoxyphosphorylamino-phenyl, 2-Chlor-4-cyano-5-ethoxycarbonyloxy-phenyl, 2-Fluor-4-cyano-5-diethoxyphosphorylmethoxyphenyl, 2-Fluor-4-cyano-5-diethylaminooxy-phenyl, 2-Fluor-4-cyano-5-dimethoxymethylenamino-phenyl, 2-Chlor-4-cyano-5-ethoxymethylenanimo-phenyl, 2-Fluor-4-cyano-5-(2-chlor-ethoxycarbonyloxy)-phenyl, 2-Chlor-4-cyano-5-dimethylaminomethylenamino-phenyl, 2-Chlor-4-cyano-5-(2-carboxy-2-chlor-ethyl)-phenyl, 2-Fluor-4-cyano-5-(2-carboxy-2-chlor-ethyl)-phenyl, 2-Fluor-4-cyano-5-i-butoxy-phenyl, 2-Chlor-4-cyano-5-i-butoxy-phenyl, 2-Chlor-4-cyano-5-(2-methoxy-ethoxy)-phenyl, 2-Fluor-4-Chlor-5-(2-methoxy-ethoxy)-phenyl, 2-Fluor-4-Chlor-5-i-butoxy-phenyl.

### Gruppe 2

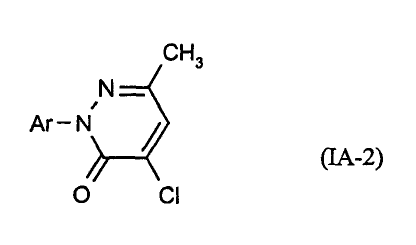

Ar hat hierbei beispielhaft die oben in Gruppe 1 aufgeführten Bedeutungen.

### Gruppe 3

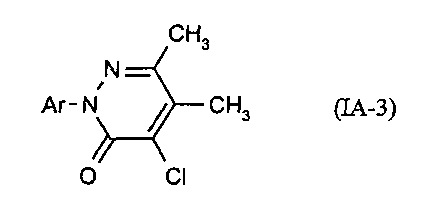

Ar hat hierbei beispielhaft die oben in Gruppe 1 aufgeführten Bedeutungen.

### Gruppe 4

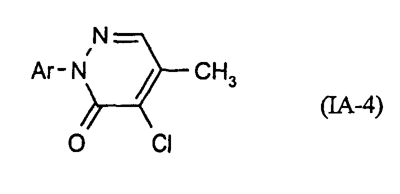

Ar hat hierbei beispielhaft die oben in Gruppe 1 aufgeführten Bedeutungen.

### Gruppe 5

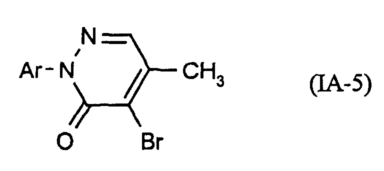

Ar hat hierbei beispielhaft die oben in Gruppe 1 aufgeführten Bedeutungen.

### Gruppe 6

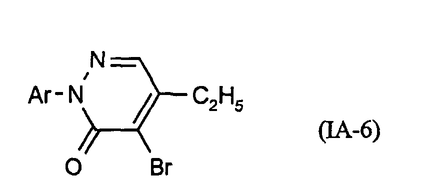

Ar hat hierbei beispielhaft die oben in Gruppe 1 aufgeführten Bedeutungen.

### Gruppe 7

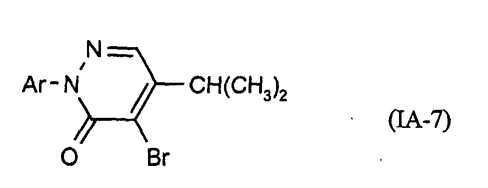

Ar hat hierbei beispielhaft die oben in Gruppe 1 aufgeführten Bedeutungen.

### Gruppe 8

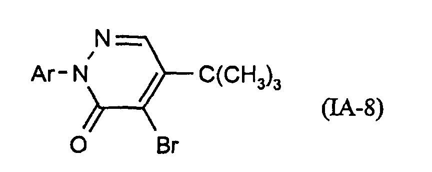

Ar hat hierbei beispielhaft die oben in Gruppe 1 aufgeführten Bedeutungen.

### Gruppe 9

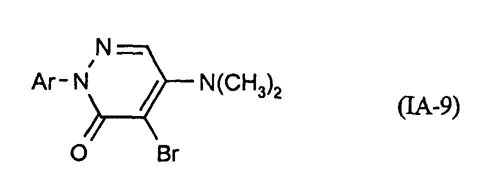

Ar hat hierbei beispielhaft die oben in Gruppe 1 aufgeführten Bedeutungen.

### Gruppe 10

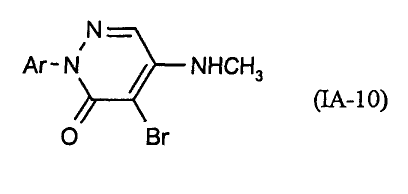

Ar hat hierbei beispielhaft die oben in Gruppe 1 aufgeführten Bedeutungen.

### Gruppe 11

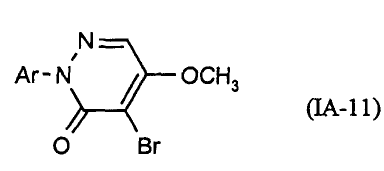

Ar hat hierbei beispielhaft die oben in Gruppe 1 aufgeführten Bedeutungen.

### Gruppe 12

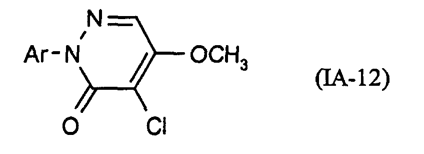

Ar hat hierbei beispielhaft die oben in Gruppe 1 aufgeführten Bedeutungen.

### Gruppe 13

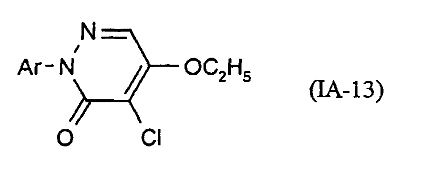

Ar hat hierbei beispielhaft die oben in Gruppe 1 aufgeführten Bedeutungen.

### Gruppe 14 A

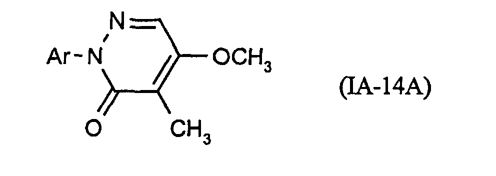

Ar hat hierbei beispielhaft die oben in Gruppe 1 aufgeführten Bedeutungen.

### Gruppe 14 B

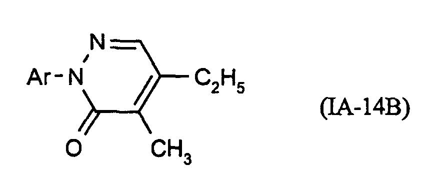

### Ar hat hierbei beispielhaft die oben in Gruppe 1 aufgeführten Bedeutungen

### Gruppe 15

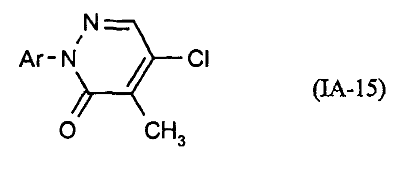

Ar hat hierbei beispielhaft die oben in Gruppe 1 aufgeführten Bedeutungen.

### Gruppe 16

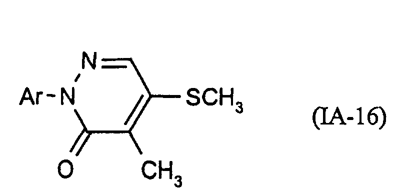

Ar hat hierbei beispielhaft die oben in Gruppe 1 aufgeführten Bedeutungen.

### Gruppe 17

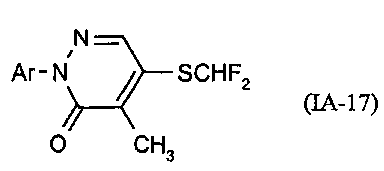

Ar hat hierbei beispielhaft die oben in Gruppe 1 aufgeführten Bedeutungen.

### Gruppe 18

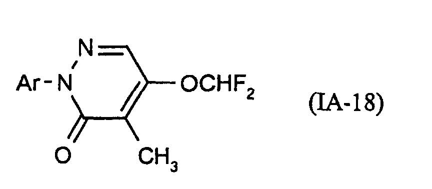

Ar hat hierbei beispielhaft die oben in Gruppe 1 aufgeführten Bedeutungen.

### Gruppe 19

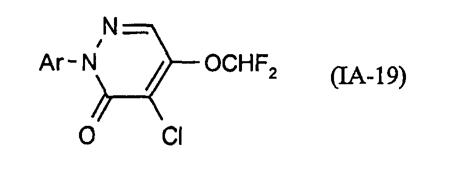

Ar hat hierbei beispielhaft die oben in Gruppe 1 aufgeführten Bedeutungen.

### Gruppe 20

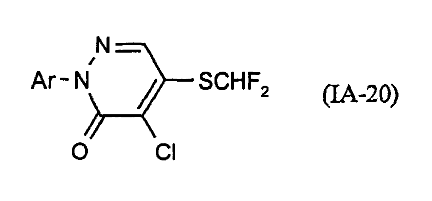

Ar hat hierbei beispielhaft die oben in Gruppe 1 aufgeführten Bedeutungen.

### Gruppe 21

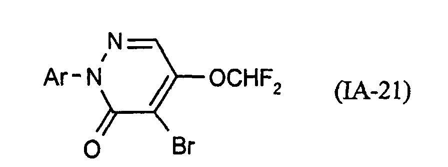

Ar hat hierbei beispielhaft die oben in Gruppe 1 aufgeführten Bedeutungen.

### Gruppe 22

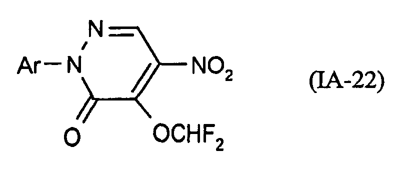

Ar hat hierbei beispielhaft die oben in Gruppe 1 aufgeführten Bedeutungen.

### Gruppe 23

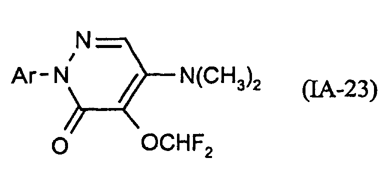

Ar hat hierbei beispielhaft die oben in Gruppe 1 aufgeführten Bedeutungen.

### Gruppe 24

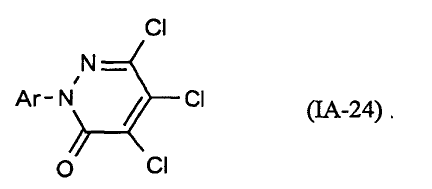

Ar hat hierbei beispielhaft die oben in Gruppe 1 aufgeführten Bedeutungen.

### Gruppe 25

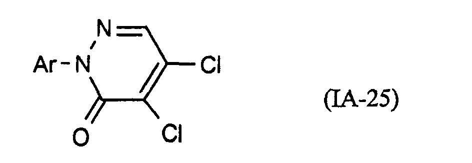

Ar hat hierbei beispielhaft die oben in Gruppe 1 aufgeführten Bedeutungen.

### Gruppe 26

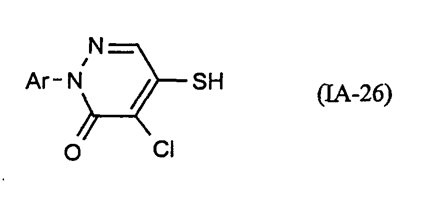

Ar hat hierbei beispielhaft die oben in Gruppe 1 aufgeführten Bedeutungen.

### Gruppe 27

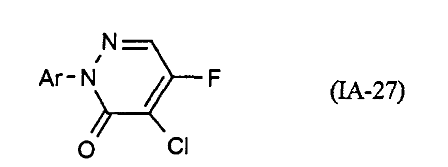

Ar hat hierbei beispielhaft die oben in Gruppe 1 aufgeführten Bedeutungen.

### Gruppe 28

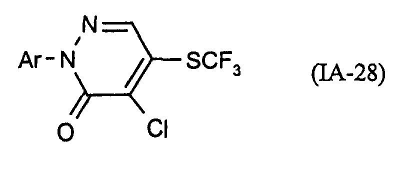

Ar hat hierbei beispielhaft die oben in Gruppe 1 aufgeführten Bedeutungen.

### Gruppe 29

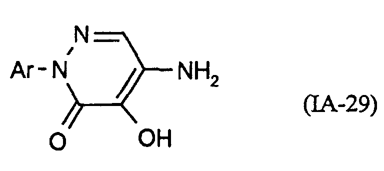

Ar hat hierbei beispielhaft die oben in Gruppe 1 aufgeführten Bedeutungen.

### Gruppe 30

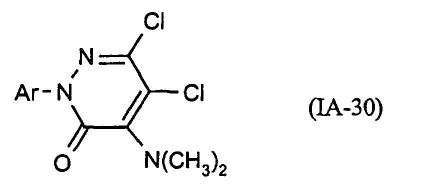

Ar hat hierbei beispielhaft die oben in Gruppe 1 aufgeführten Bedeutungen.

### Gruppe 31

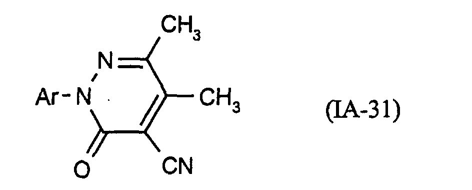

Ar hat hierbei beispielhaft die oben in Gruppe 1 aufgeführten Bedeutungen.

### Gruppe 32

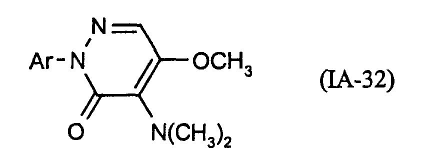

Ar hat hierbei beispielhaft die oben in Gruppe 1 aufgeführten Bedeutungen.

### Gruppe 33

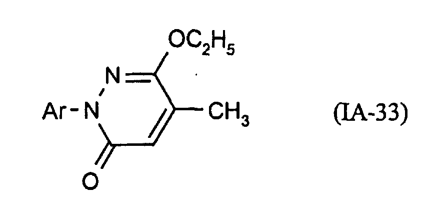

Ar hat hierbei beispielhaft die oben in Gruppe 1 aufgeführten Bedeutungen.

### Gruppe 34

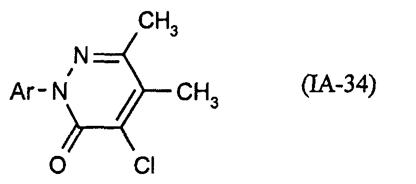

Ar hat hierbei beispielhaft die oben in Gruppe 1 aufgeführten Bedeutungen.

### Gruppe 35

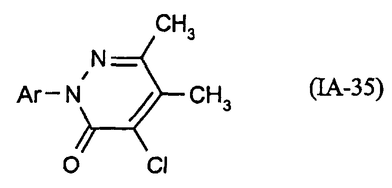

Ar hat hierbei beispielhaft die oben in Gruppe 1 aufgeführten Bedeutungen.

### Gruppe 36

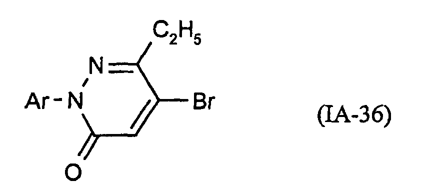

Ar hat hierbei beispielhaft die oben in Gruppe 1 aufgeführten Bedeutungen.

### Gruppe 37

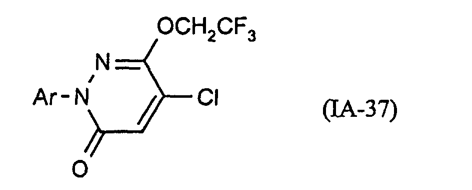

Ar hat hierbei beispielhaft die oben in Gruppe 1 aufgeführten Bedeutungen.

### Gruppe 38

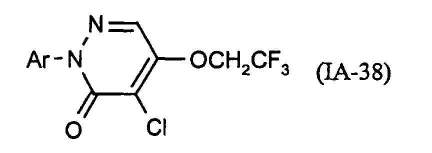

Ar hat hierbei beispielhaft die oben in Gruppe 1 aufgeführten Bedeutungen.

### Gruppe 39

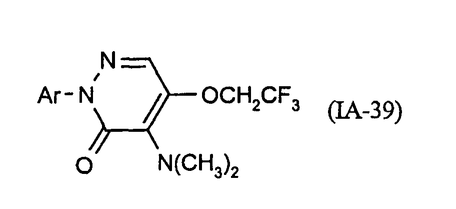

Ar hat hierbei beispielhaft die oben in Gruppe 1 aufgeführten Bedeutungen.

### Gruppe 40

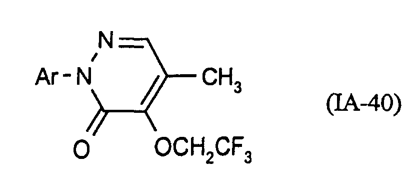

Ar hat hierbei beispielhaft die oben in Gruppe 1 aufgeführten Bedeutungen.

### Gruppe 41

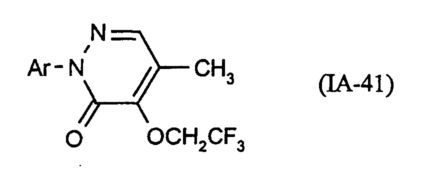

Ar hat hierbei beispielhaft die oben in Gruppe 1 aufgeführten Bedeutungen.

Verwendet man beispielsweise 4-Fluor-6-methyl-pyridazin-3-on und 4,5-Difluor-2-methoxy-benzonitril als Ausgangsstoffe, so kann der Reaktionsablauf beim erfindungsgemäßen Verfahren (a) durch das folgende Formelschema skizziert werden:

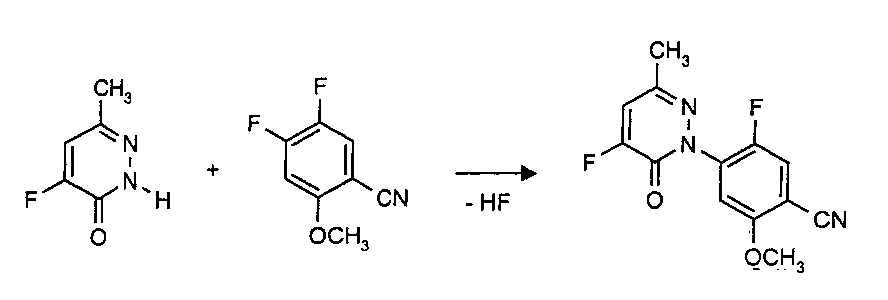

Verwendet man beispielsweise 4,4,4-Trichlor-3-methyl-crotonaldehyd und 2,4-Dichlor-5-trifluormethyl-phenylhydrazin als Ausgangsstoffe, so kann der Reaktionsablauf beim erfindungsgemäßen Verfahren (b) durch das folgende Formelschema skizziert werden:

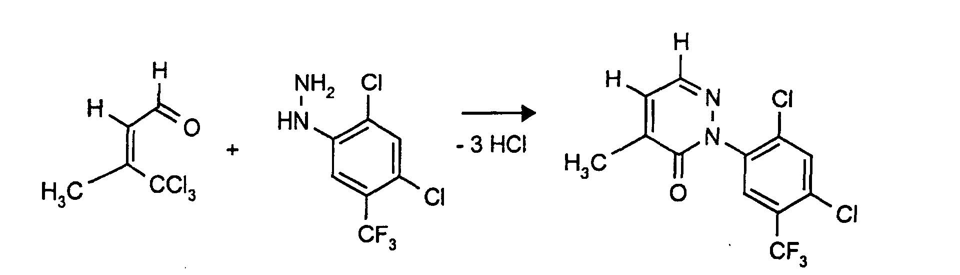

Verwendet man beispielsweise Mucochlorsäure-N-(4-brom-2-fluor-5-methoxycarbonyl-phenyl)-hydrazon als Ausgangsstoff, so kann der Reaktionsablauf beim erfindungsgemäßen Verfahren (c) durch das folgende Formelschema skizziert werden:

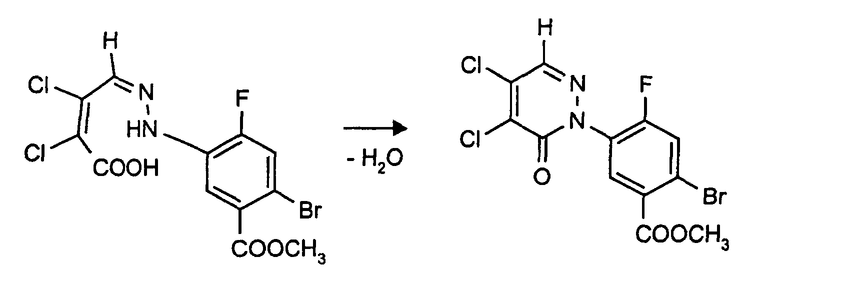

Verwendet man beispielsweise 2-(2,4-Dichlor-phenyl)-4,5,6-trichlor-pyridazin-3-on und Salpetersäure als Ausgangsstoffe, so kann der Reaktionsablauf beim erfindungsgemäßen Verfahren (d) durch das folgende Formelschema skizziert werden:

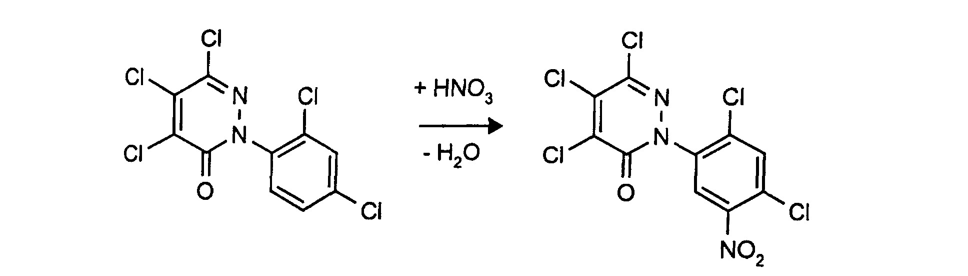

Die beim erfindungsgemäßen Verfahren (a) zur Herstellung der Verbindungen der Formel (I) als Ausgangsstoffe zu verwendenden Halogenarene sind durch die Formel (II) allgemein definiert. In der Formel (II) haben R¹, R² und R³ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R¹, R² und R³ angegeben wurden; X¹ steht vorzugsweise für Fluor, Chlor oder Brom, insbesondere für Fluor.

Die Ausgangsstoffe der Formel (II) sind bekannt und/oder können nach bekannten Verfahren hergestellt werden (vgl. EP 191 181, EP 370 332, EP 431 373, EP 441 004).

Die beim erfindungsgemäßen Verfahren (a) weiter als Ausgangsstoffe zu verwendenden Pyridazinone sind durch die Formel (III) allgemein definiert. In der Formel (III) haben R⁴, R⁵ und R⁶ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R⁴, R⁵ und R⁶ angegeben wurden.

Die Ausgangsstoffe der Formel (III) sind bekannt und/oder können nach bekannten Verfahren hergestellt werden (vgl. J. Chem. Soc. 1947, 239; Angew. Chem. 77 (1965), 282; Monatsh. Chem. 120 (1989), 329).

Die beim erfindungsgemäßen Verfahren (b) zur Herstellung der Verbindungen der Formel (I) als Ausgangsstoffe zu verwendenden Arylhydrazine sind durch die Formel (IV) allgemein definiert. In der Formel (IV) haben R¹, R² und R³ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R¹, R² und R³ angegeben wurden.

Die Ausgangsstoffe der Formel (IV) sind bekannt und/oder können nach bekannten Verfahren hergestellt werden (vgl. EP 370 332).

Die beim erfindungsgemäßen Verfahren (b) weiter als Ausgangsstoffe zu verwendenden β-Trihalomethyl-enone sind durch die Formel (V) allgemein definiert. In der Formel (V) haben R⁴, R⁵ und R⁶ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R⁴, R⁵ und R⁶ angegeben wurden; X² steht vorzugsweise für Fluor, Chlor oder Brom, insbesondere für Chlor.

Die Ausgangsstoffe der Formel (V) sind bekannt und/oder können nach bekannten Verfahren hergestellt werden (vgl. DE 2 706 700).

Die beim erfindungsgemäßen Verfahren (c) zur Herstellung der Verbindungen der Formel (I) als Ausgangsstoffe zu verwendenden Hydrazoncarbonsäuren sind durch die Formel (VI) allgemein definiert. In der Formel (VI) haben R¹, R², R³, R⁴, R⁵ und R⁶ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R¹, R², R³, R⁴, R⁵ und R⁶ angegeben wurden.

Die Ausgangsstoffe der Formel (VI) sind noch nicht aus der Literatur bekannt; sie sind als neue Stoffe Gegenstand der vorliegenden Anmeldung.

Man erhält die neuen Hydrazoncarbonsäuren der allgemeinen Formel (VI), wenn man Arylhydrazine der allgemeinen Formel (IV) 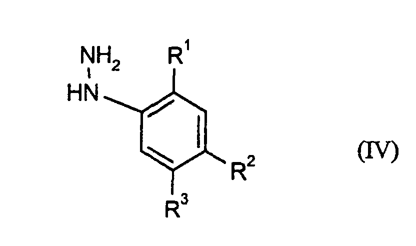 in welcher
R¹, R² und R³ die oben angegebenen Bedeutungen haben,
mit β-Carboxy-enonen der allgemeinen Formel (VII) 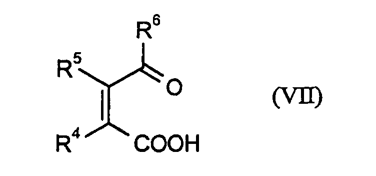 in welcher
R⁴, R⁵ und R⁶ die oben angegebenen Bedeutungen haben,
gegebenenfalls in Gegenwart eines Verdünnungsmittels, wie z.B. Ethanol, und gegebenenfalls in Gegenwart eines Reaktionshilfsmittels, wie z.B. p-Toluolsulfonsäure, bei Temperaturen zwischen 0°C und 100°C umsetzt (vgl. die Herstellungsbeispiele).

Die beim erfindungsgemäßen Verfahren (d) zur Herstellung der Verbindungen der Formel (I) als Ausgangsstoffe zu verwendenden 2,4-disubstituierten Phenylpyridazinone sind durch die Formel (Ia) allgemein definiert. In der Formel (Ia) haben R¹, R², R⁴, R⁵ und R⁶ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R¹, R², R⁴, R⁵ und R⁶ angegeben wurden.

Die Ausgangsstoffe der Formel (Ia) sind bekannt und/oder können nach bekannten Verfahren hergestellt werden (vgl. die erfindungsgemäßen Verfahren (a) bis (c)).

Die erfindungsgemäßen Verfahren (a), (b), (c) und (d) zur Herstellung der Verbindungen der Formel (I) werden vorzugsweise in Gegenwart eines Verdünnungsmittels durchgeführt. Als Verdünnungsmittel kommen im Allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether, Ethyl-t-butylether, Methyl-t-pentylether (MTBE), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -i-propylester, -n-, -i- oder -sbutylester; Sulfoxide, wie beispielsweise Dimethylsulfoxid; Alkanole, wie beispielsweise Methanol, Ethanol, n- oder i-Propanol, n-, i-, s- oder t-Butanol, Ethylenglycolmonomethylether oder -monoethylether, Diethylenglycol-monomethylether oder -monoethylether, deren Gemische mit Wasser oder reines Wasser. Beim erfindungsgemäßen Verfahren (b) kann vorteilhaft auch Essigsäure als Verdünnungsmittel eingesetzt werden.

Die erfindungsgemäßen Verfahren (a) und (b) zur Herstellung der Verbindungen der Formel (I) werden vorzugsweise in Gegenwart eines geeigneten Reaktionshilfsmittels durchgeführt. Als Reaktionshilfsmittel kommen im Allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- öder Calciumamid, Natrium-, Kalium- oder Calcium-carbonat, Natrium-, Kalium- oder Calciumhydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylarnin, N,N-Dimethyl-anilin, N,N-Dimethylbenzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylaminopyridin, N-Methyl-piperidin, 1,4-Diazabicyclo[2,2,2]-octan (DABCO), 1,5-Diazabicyclo[4,3,0]-non-5-en (DBN), und 1,8-Diazabicyclo[5,4,0]-undec-7-en (DBU).

Das erfindungsgemäße Verfahren (c) zur Herstellung der Verbindungen der Formel (I) wird in Gegenwart eines wasser-entziehenden Mittels durchgeführt. Es kommen hierbei die üblichen Dehydratisierungsmittel, wie z.B. Schwefelsäure, Methansulfonsäure, Benzolsulfonsäure, p-Toluolsulfonsäure, Acetanhydrid und Phosphor(V)-oxid in Betracht.

Das erfindungsgemäße Verfahren (d) zur Herstellung der Verbindungen der Formel (I) wird unter Verwendung eines Nitrierungsmittels durchgeführt. Es kommen hierbei die üblichen Nitrierungsmittel, wie z.B. Salpetersäure und deren Mischungen mit Nitrierungshilfsmitteln, wie z.B. Schwefelsäure, in Betracht.

Die Reaktionstemperaturen können bei der Durchführung der erfindungsgemäßen Verfahren (a), (b), (c) und (d) in einem größeren Bereich variiert werden. Im Allgemeinen arbeitet man bei Temperaturen zwischen -20°C und +200°C, vorzugsweise zwischen 0°C und 150°C, insbesondere zwischen 10°C und 120°C.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im Allgemeinen zwischen 0,1 bar und 10 bar - durchzuführen.

Zur Durchführung der erfindungsgemäßen Verfahren (a), (b), (c) und (d) werden die Ausgangsstoffe im Allgemeinen in angenähert äquimolaren Mengen eingesetzt. Es ist jedoch auch möglich, eine der Komponenten in einem größeren Überschuß zu verwenden. Die Umsetzung wird im Allgemeinen in einem geeigneten Verdünnungsmittel gegebenenfalls in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofopmethyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiocarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

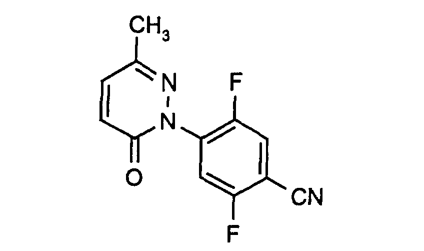

### (Verfahren (a))

20 g (0,10 mol) 3-Methyl-pyridazin-6-on-Hydrobromid und 30 g (0,22 mol) Kaliumcarbonat werden in 200 ml Dimethylsulfoxid vorgelegt und bei Raumtemperatur (ca.20°C) mit 15,7 g (0,10 mol) 2,4,5-Trifluorbenzonitril versetzt. Die Mischung wird über Nacht bei 40°C - 50°C gerührt und anschließend am Rotationsverdampfer eingeengt. Der Rückstand wird mit Wasser verrührt, abgesaugt und aus Isopropanol umkristallisiert.

Man erhält 18,5 g (76 % der Theorie) 2-(2,5-Difluor-4-cyano-phenyl)-6-methylpyridazin-3-on vom Schmelzpunkt 159°C.

### Beispiel 2

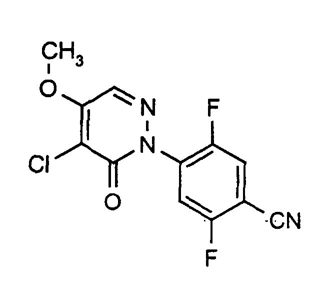

### (Verfahren (a))

5,0 g (0,03 mol) 5-Chlor-4-methoxy-pyridazin-6-on und 5,0 g Kaliumcarbonat werden in 50 ml Dimethylsulfoxid vorgelegt und bei Raumtemperatur (ca. 20°C) mit 4,7 g (0,03 mol) 2,4,5-Trifluor-benzonitril versetzt. Die Mischung wird 2 Stunden bei Raumtemperatur gerührt, auf Wasser gegeben, ausgefallenes Produkt abgesaugt, mit Wasser gewaschen und getrocknet.

Man erhält 7,7 g (86 % der Theorie) 2-(2,5-Difluor-4-cyano-phenyl)-4-chlor-5-methoxy-pyridazin-3-on vom Schmelzpunkt 182°C.

### Beispiel 3

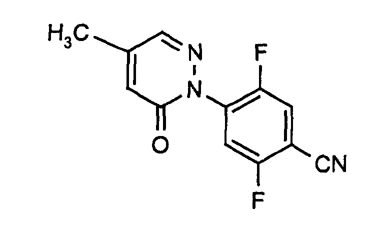

### (Verfahren (b))

Zu 8,8 g (0,047 mol) 4,4,4-Trichlor-2-methyl-crotonaldehyd, 11,5 g (0,14 mol) Natriumacetat und 20 ml Wasser in 100 ml Essigsäure werden bei Raumtemperatur (ca. 20°C) 7,9 g (0,047 mol) 2,5-Difluor-4-cyano-phenylhydrazin gegeben und die Mischung wird über Nacht bei 50°C gerührt. Die auf Raumtemperatur abgekühlte Mischung wird mit Wasser verrührt, abgesaugt und der Rückstand aus n-Hexan umkristallisiert.

Man erhält 7,2 g (62 % der Theorie) 2-(2,5-Difluor-4-cyano-phenyl)-5-methylpyridazin-3-on vom Schmelzpunkt 223°C.

### Beispiel 4

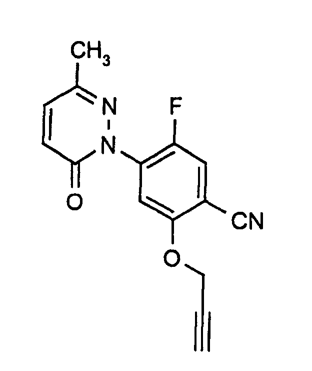

1,68 g (0,03 mol) Propargylalkohol werden in 50 ml Acetonitril mit 0,9 g (0,03 mol) 80% igem Natriumhydrid 15 Minuten bei Raumtemperatur (ca. 20°C) gerührt, anschließend mit 3,7 g (0,015 mol) 2-(2,5-Difluor-4-cyano-phenyl)-6-methyl-pyridazin-3-on versetzt, weitere 5 Stunden bei Raumtemperatur gerührt, auf Wasser gegeben, mit konz. Salzsäure angesäuert, ausgefallenes Produkt abgesaugt und aus Methanol umkristallisiert.

Man erhält 1,26 g (27 % der Theorie) 2-(2-Fluor-4-cyano-5-propargyloxy-phenyl)-6-methyl-pyridazin-3-on vom Schmelzpunkt 180°C.

### Beispiel 5

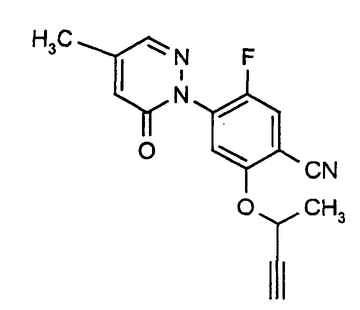

1,1 g (0,015 mol) Butin-1-ol-(3) werden in 50 ml Acetonitril mit 0,45 g (0,015 mol) 80 %igem Natriumhydrid 15 Minuten bei Raumtemperatur (ca. 20°C) gerührt, anschließend mit 2,5 g (0,01 mol) 2-(2,5-Difluor-4-cyano-phenyl)-5-methyl-pyridazin-3-on versetzt, über Nacht bei Raumtemperatur gerührt, auf Wasser gegeben, mit konz. Salzsäure angesäuert, ausgefallenes Produkt abgesaugt und getrocknet.

Man erhält 2,6 g (88 % der Theorie) 2-(2-Fluor-4-cyano-5-but-1-in-3-yl-oxy-phenyl)-5-methyl-pyridazin-3-on vom Schmelzpunkt 108°C.

### Beispiel 6

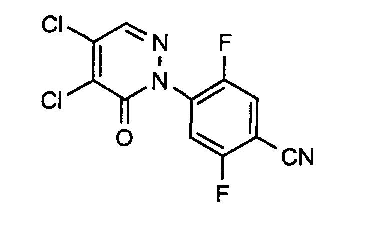

Zu 53 g (0,166 mol) 2-(2,5-Difluor-4-carboxamido-phenyl)-4,5-dichlor-pyridazin-3-on in 300 ml Toluol und 2 ml Dimethylformamid werden bei 70°C innerhalb einer Stunde 39 g (0,30 mol) Thionylchlorid tropfenweise gegeben. Die Mischung wird ca. 90 Minuten bei Rückflußtemperatur gerührt, heiß filtriert, kalt ausgefallenes Produkt abgesaugt, mit Toluol gewaschen und getrocknet.

Man erhält 35 g (70 % der Theorie) 2-(2,5-Difluor-4-cyano-phenyl)-4,5-dichlorpyridazin-3-an vom Schmelzpunkt 206°C.

### Beispiel 7

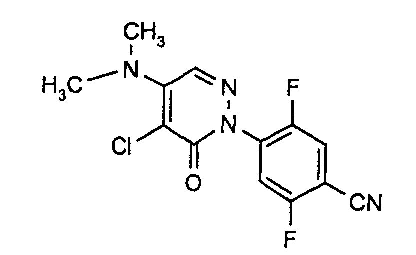

Eine Mischung aus 4,22 g (0,014 mol) 2-(2,5-Difluor-4-cyano-phenyl)-4,5-dichlorpyridazin-3-on, 1,38 g (0,017 mol) Dimethylamin-hydrochlorid und 3,1 g (0,03 mol) Triethylamin wird in 50 ml Acetonitril über Nacht (ca. 15 Stunden) bei Raumtemperatur (ca. 20°C) gerührt, dann auf Wasser gegeben, abgesaugt, mit Wasser und anschließend mit Isopropanol gewaschen.

Man erhält 3,35 g (77 % der Theorie) 2-(2,5-Difluor-4-cyano-phenyl)-4-chlor-5-dimethylamino-pyridazin-3-on vom Schmelzpunkt 158°C.

### Beispiel 8

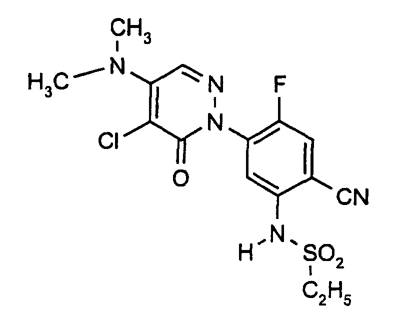

1,55 g (5 mmol) 2-(2,5-Difluor-4-cyano-phenyl)-4-chlor-5-dimethylamino-pyridazin-3-on, 0,9 g (6 mmol) Kaliumcarbonat und 0,65 g (6 mmol) Ethansulfonamid werden in 10 ml Dimethylsulfoxid 8 Stunden bei 110°C gerührt. Die Mischung wird eingeengt, mit Wasser verrührt, mit konz. Salzsäure angesäuert, ausgefallenes Produkt abgesaugt und der Rückstand mittels Säulen-chromatographie (Laufinittel: Cyclohexan/Essigester 1:1) gereinigt.

Man erhält 0,6 g (30 % der Theorie) 2-(4-Cyano-2-fluor-5-ethylsulfonylamino-phenyl)-4-chlor-5-dimethylamino-pyridazin-3-on vom Schmelzpunkt 206°C.

### Beispiel 9

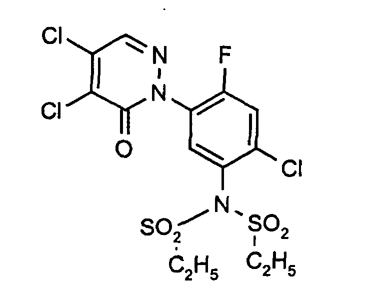

12 g (0,04 mol) 2-(2-Fluor-4-Chlor-5-amino-phenyl)-4,5-dichlor-pyridazin-3-on und 24 g (0,24 mol) Triethylamin werden in 300 ml Methylenchlorid auf-10°C abgekühlt, innerhalb 15 Minuten 31 g (0,24 mol) Ethansulfonsäurechlorid zugetropft, die Mischung 30 Minuten bei -10°C, 3 Stunden bei Raumtemperatur gerührt, mit Wasser versetzt, die organische Phase abgetrennt, über Natriumsulfat getrocknet, eingeengt und der Rückstand aus Isopropanol umkristallisiert.

Man erhält 14,9 g (76 % der Theorie) 2-[2-Fluor-4-chlor-5-(bis-ethylsulfonyl)-aminophenyl]-4,5-dichlor-pyridazin-3-on vom Schmelzpunkt 188°C.

Analog Beispiel 1 bis 14 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

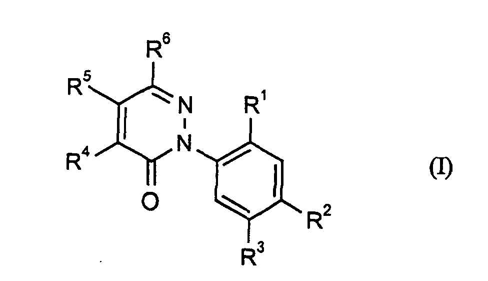

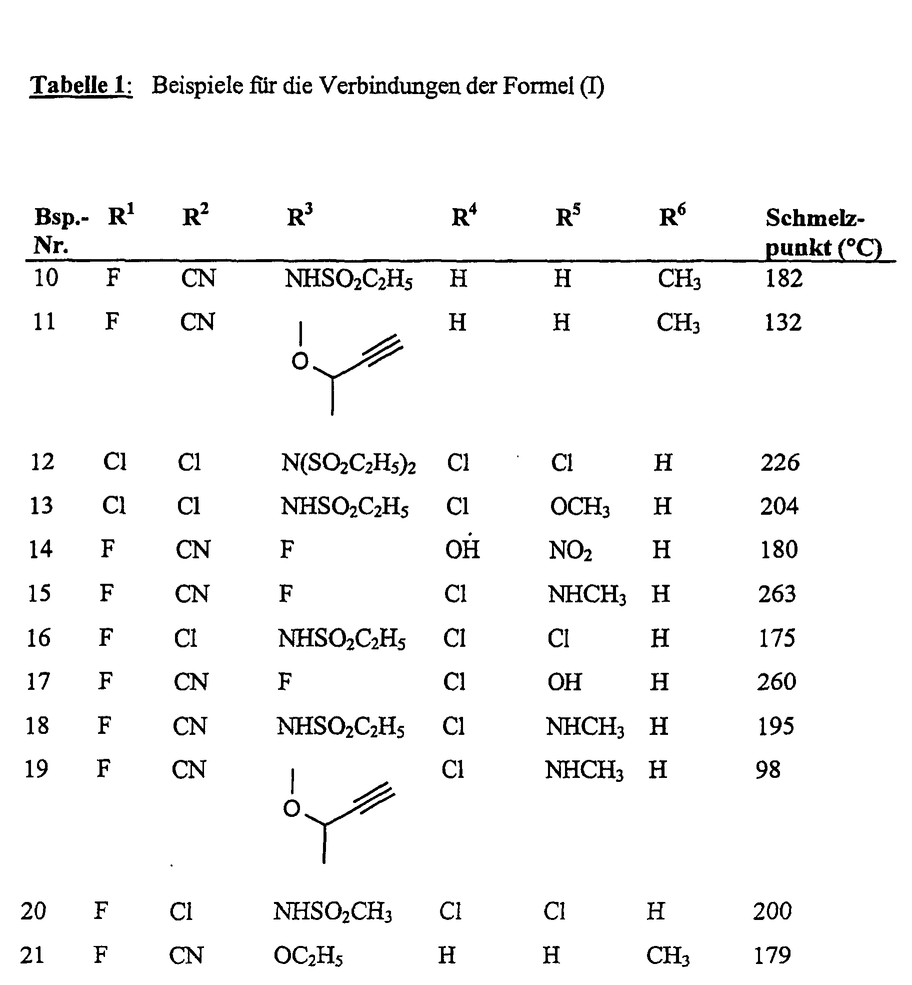

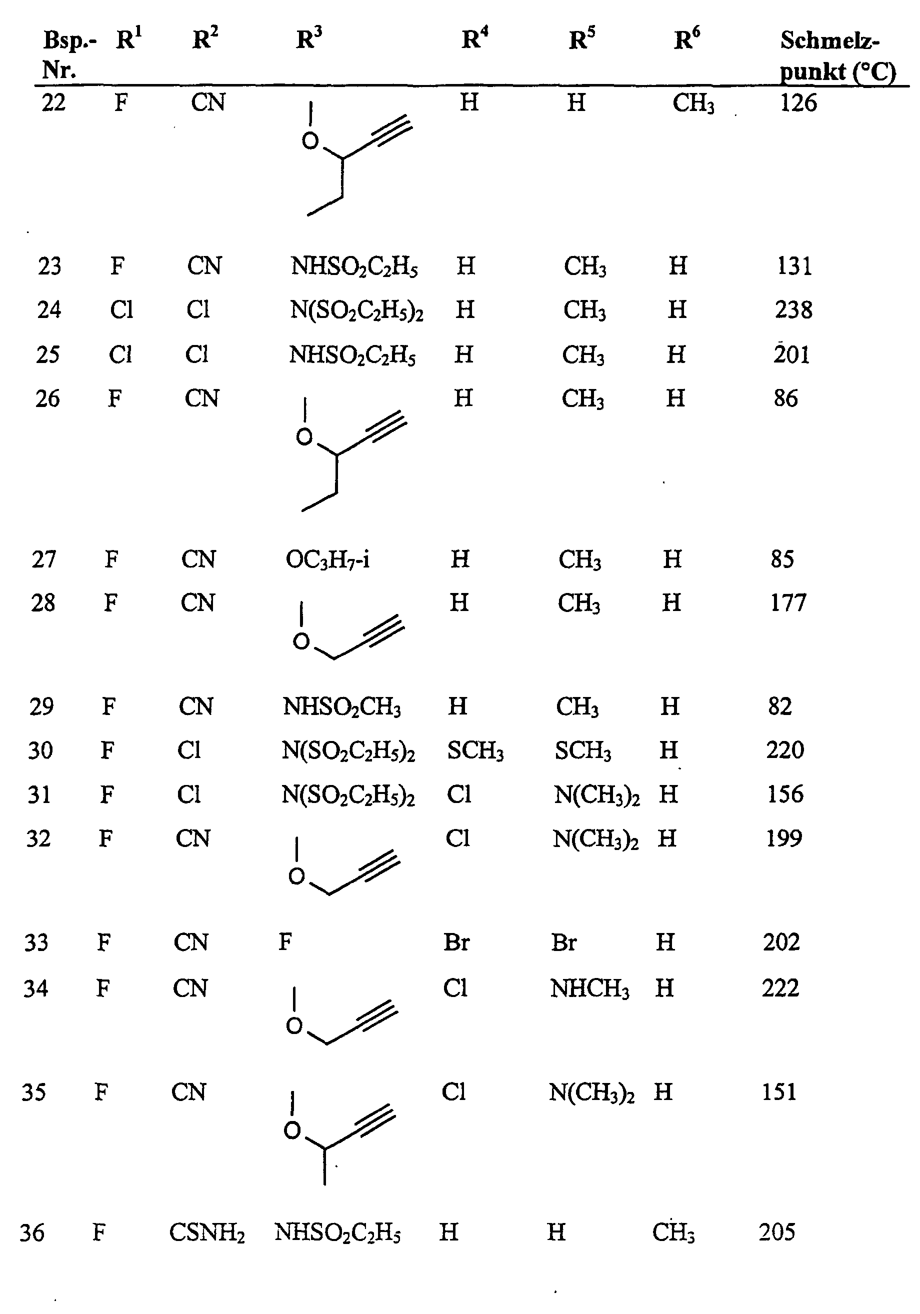

### Ausgangsstoffe der Formel (Ia):

### Beispiel (Ia-1)

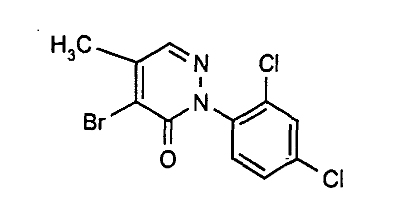

Zu 12,5 g (47 mmol) 4,4,4-Trichlor-3-brom-2-methyl-crotonaldehyd, 11,5 g (140 mmol) Natriumacetat und 40 ml Wasser in 150 ml Essigsäure werden bei Raumtemperatur (ca.20°C) 10,1 g (47 mmol) 2,4-Dichlor-phenyl-hydrazin-Hydrochlorid gegeben und die Mischung wird über Nacht bei 40°C gerührt. Die auf Raumtemperatur abgekühlte Mischung wird mit Wasser verrührt und abgesaugt.

Man erhält 7,5 g (48 % der Theorie) 2-(2,4-Dichlor-phenyl)-4-brom-5-methyl-pyridazin-3-on vom Schmelzpunkt 108°C.

### Beispiel (Ia-2)

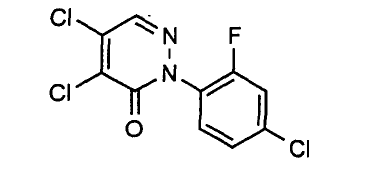

56,2 g (0,35 mol) 2-Fluor-4-chlor-phenyl-hydrazin, 67,6 g Mucochlorsäure und 2 g p-Toluolsulfonsäure werden in 700 ml Ethanol 12 Stunden bei Rückflußtemperatur gerührt, kalt ausgefallenes Produkt abgesaugt, mit Ethanol gewaschen und getrocknet.

Man erhält 69,8 g (68 % der Theorie) 2-(2-Fluor-4-chlor-phenyl)-4,5-dichlor-pyridazin-3-on vom Schmelzpunkt 158°C.

### Ausgangsstoffe der Formel (VI):

### Beispiel (VI-1)

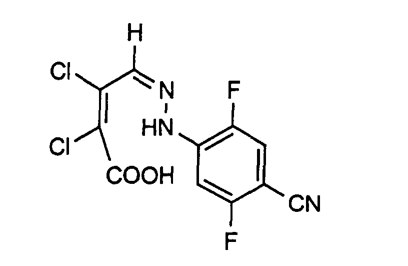

84,5 g (0,5 mol) 2,5-Difluor-4-cyano-phenyl-hydrazin werden in 700 ml Ethanol vorgelegt und unter Rühren 84,5 g (0,6 mol) Mucochlorsäure eingetragen. Die langsam einsetzende exotherme Reaktion erreicht nach 30 Minuten 50°C. Nach abklingender Reaktion werden 1 g p-Toluolsulfonsäure zugesetzt und 12 Stunden bei Rückflusstemperatur gerührt, der ausgefallene Feststoff wird kalt abgesaugt und mit Ethanol gewaschen.

Man erhält 133 g (83 % der Theorie) Mucochlorsäure-2,5-difluor-4-cyano-phenylhydrazon vom Schmelzpunkt > 260°C.

### Anwendungsbeispiele:

In den Anwendungsbeispielen werden die nachstehend aufgeführten Verbindungen (A) und (B) als Vergleichssubstanzen herangezogen.

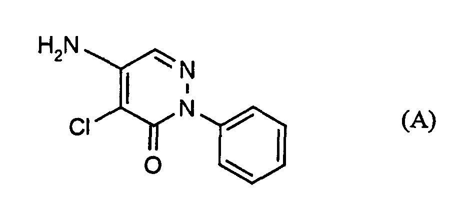

5-Amino-4-chlor-2-phenyl-pyridazin-3-on (bekannt aus DE 1105232);

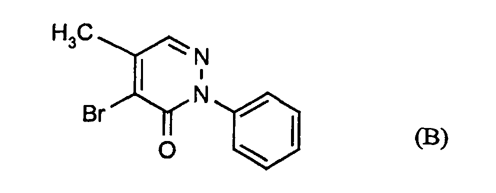

4-Brom-5-methyl-2-phenyl-pyridazin-3-an (bekannt aus DE 2706700).

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen bzw. gespritzt. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 3, 7, 8, 15, 16, 25, 29, 31, 34, 35, 36 und 41 bei guter Verträglichkeit gegenüber Kulturpflanzen, wie z.B. Sonnenblumen (0-20 %) und Mais (0-10 %) und einer Aufwandmenge zwischen 125 und 500 g/ha, starke Wirkung gegen Unkräuter wie z.B. Abutilon (100 %), Chenopodium (90-100 %), Galinsoga (80-100 %) und Solanum (60-100 %).

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, dass die jeweils gewünschten Wirkstoffmengen pro Flächeneinheit ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiele 8, 15, 16, 31, 35, 37 und 41 bei weitgehend guter Verträglichkeit gegenüber Kulturpflanzen, wie z.B. Weizen (0-20 %) und einer Aurwandmenge zwischen 60 und 250 g/ha, starke Wirkung gegen Unkräuter wie Amaranthus (60-100 %), Polygonum (80-100 %) und Veronica (90-100%).

## Patentansprüche

1. Phenylpyridazinone der allgemeinen Formel (I) 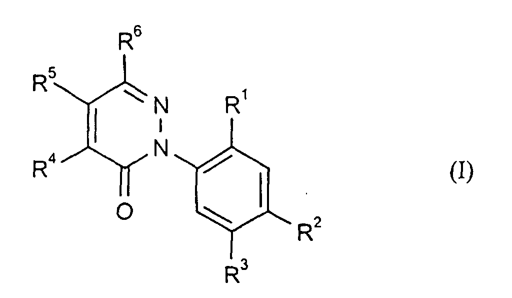 in welcher
R¹ für Fluor, Chlor oder Brom steht,
R² für Cyano, Carbamoyl, Thiocarbamoyl, Fluor, Chlor, Brom, oder für jeweils gegebenenfalls durch Fluor und/oder Chlor substituiertes Alkyl, Alkoxy oder Alkylthio mit jeweils 1 oder 2 Kohlenstoffatomen steht,
R³ für die Gruppierung -A¹-A²-A³ steht,
in welcher
A¹ für eine Einfachbindung, für Sauerstoff oder die Gruppierung -N-A⁴- steht, worin A⁴ für Wasserstoff, Hydroxy, C₁-C₄-Alkyl, C₃-C₄-Alkenyl, C₃-C₄-Alkinyl, C₁-C₄-Alkoxy, Phenyl, C₁-C₄-Alkylsulfonyl oder Phenylsulfonyl steht,
A² für eine Einfachbindung oder für gegebenenfalls durch Fluor, Chlor oder Brom substituiertes C₁-C₆-Alkandiyl steht,
A³ für Fluor, Chlor, Brom steht,
A³ weiterhin für jeweils gegebenenfalls durch Fluor, Chlor oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy(thio)phosphoryl mit jeweils 1 bis 6 Kohlenstoffatomen in den Alkylgruppen steht,
A³ weiterhin für jeweils gegebenenfalls durch Fluor oder Chlor substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen steht, und
R⁴, R⁵ und R⁶ gleich oder verschieden sind und für Wasserstoff, Cyano, Thiocarbamoyl, Nitro, Hydroxy, Mercapto, Amino, Fluor, Chlor, Brom, oder für jeweils gegebenenfalls durch Fluor und/oder Chlor substituiertes Alkyl, Alkoxy, Alkylthio, Alkylamino oder Dialkylamino mit jeweils 1 bis 4 Kohlenstoffatomen in den Alkylgruppen stehen,
unter Ausnahme der Verbindungen 2-(2,4,5-Trichlor-phenyl)-pyridazin-3-on und 4-Chlor-5-dimethylamino-2-(4-chlor-2-fluor-5-propargyloxy-phenyl)-pyridazin-3-on.

2. Phenylpyridazinone gemäß Anspruch 1, **dadurch gekennzeichnet, daß**
R¹ für Fluor oder Chlor steht,
R² für Cyano, Thiocarbamoyl, Fluor, Chlor, Brom, Methyl oder Trifluormethyl steht,
R³ für die Gruppierung -A¹-A²-A³ steht,
in welcher
A¹ für eine Einfachbindung, für Sauerstoff oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl oder Ethylsulfonyl steht,
A² für eine Einfachbindung oder für Methylen, Ethan-1,1-diyl, Ethan-1,2-diyl, Propan-1,1-diyl, Propan-1,2-diyl oder Propan-1,3-diyl steht,
A³ für Fluor, Chlor, Brom steht,
A³ weiterhin für jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, n-, i-, s- oder t-Pentyloxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Methoxycarbonyl, Ethoxycarbonyl, n- oder i-Propoxycarbonyl, Dimethoxyphosphoryl, Diethoxyphosphoryl, Dipropoxyphosphoryl oder Diisopropoxyphosphoryl steht, und
A³ weiterhin für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl steht, und
R⁴, R⁵ und R⁶ gleich oder verschieden sind und für Wasserstoff, Cyano, Thiocarbamoyl, Nitro, Hydroxy, Mercapto, Amino, Fluor, Chlor, Brom, oder für jeweils gegebenenfalls durch Fluor und/oder Chlor substituiertes Methyl, Ethyl, n- oder i-Propyl, n-, i-, s- oder t-Butyl, Methoxy, Ethoxy, Methylthio, Ethylthio, Methylamino, Ethylamino oder Dimethylamino stehen.

3. Verfahren zur Herstellung der Phenylpyridazinone gemäß Anspruch 1 oder 2,
**dadurch gekennzeichnet, daß** man
(a) Halogenarene der allgemeinen Formel (II) 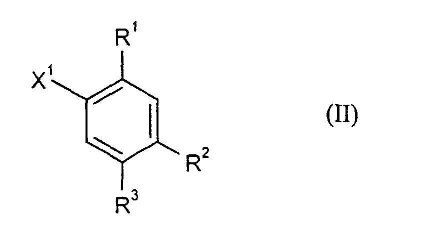 in welcher
R¹, R² und R³ die in Anspruch 1 oder 2 angegebenen Bedeutungen haben und
X¹ für Halogen steht,
mit Pyridazinonen der allgemeinen Formel (III) 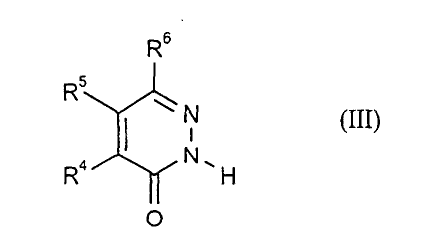 in welcher
R⁴, R⁵ und R⁶ die in Anspruch 1 oder 2 angegebenen Bedeutungen haben,
- oder mit Säureaddukten oder Alkalimetallsalzen von Verbindungen der Formel (III) -
gegebenenfalls in Gegenwart eines Reaktionshilfsmittels und gegebenenfalls in Gegenwart eines Verdünnungsmittels umsetzt,
oder daß man
(b) Arylhydrazine der allgemeinen Formel (IV) 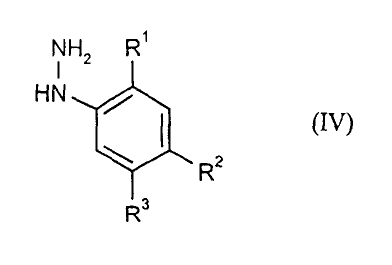 in welcher
R¹, R² und R³ die oben angegebenen Bedeutungen haben,
mit β-Trihalomethyl-enonen der allgemeinen Formel (V) 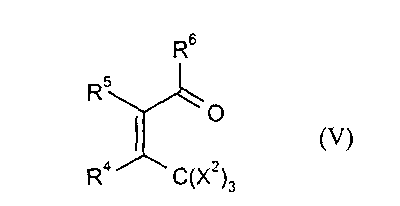 in welcher
R⁴, R⁵ und R⁶ die oben angegebenen Bedeutungen haben und
X² für Halogen steht,
gegebenenfalls in Gegenwart eines Reaktionshilfsmittels und gegebenenfalls in Gegenwart eines Verdünnungsmittels umsetzt,
oder daß man
(c) Hydrazoncarbonsäuren der allgemeinen Formel (VI) 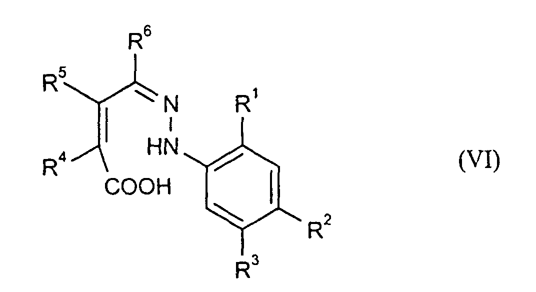 in welcher
R¹, R², R³, R⁴, R⁵ und R⁶ die oben angegebenen Bedeutungen haben, cyclisierend kondensiert, d.h. mit einem wasser-entziehenden Mittel umsetzt, oder daß man
(d) 2,4-disubstituierte Phenylpyridazinone der allgemeinen Formel (Ia) 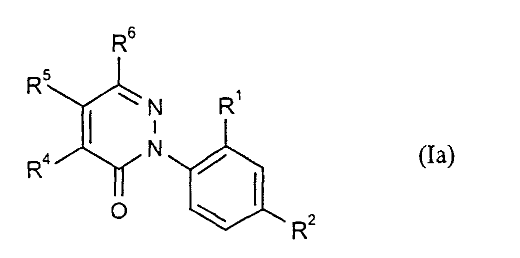
in welcher
R¹, R², R⁴, R⁵ und R⁶ die oben angegebenen Bedeutungen haben,
nitriert, d.h. mit einem Nitrierungsmittel umsetzt.

4. Herbizide Mittel, **gekennzeichnet durch** einen Gehalt an mindestens einem Phenylpyridazinon gemäß Anspruch 1 oder 2.

5. Verfahren zur Bekämpfung von unerwünschten Pflanzen, **dadurch gekennzeichnet, daß** man Phenylpyridazinone gemäß Anspruch 1 oder 2 auf unerwünschte Pflanzen und/oder ihren Lebensraum einwirken läßt.

6. Verwendung von Phenylpyridazinonen gemäß Anspruch 1 oder 2 zur Bekämpfung von unerwünschten Pflanzen.

7. Verfahren zur Herstellung von herbiziden Mitteln, **dadurch gekennzeichnet, daß** man Phenylpyridazinone gemäß Anspruch 1 oder 2 mit Streckmitteln und/oder oberflächenaktiven Substanzen vermischt.

8. Hydrazoncarbonsäuren der allgemeinen Formel (VI) 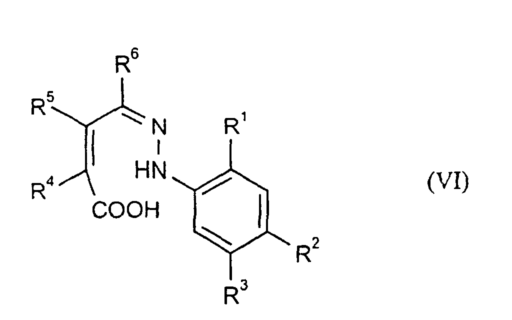 in welcher
R¹, R², R³, R⁴, R⁵ und R⁶ die in Anspruch 1 oder 2 angegebenen Bedeutungen haben.

## Claims

1. Phenylpyridazinones of the general formula (I) 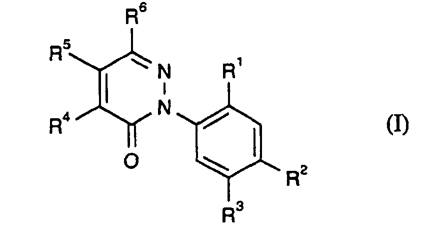 in which
R¹ represents fluorine, chlorine or bromine,
R² represents cyano, carbamoyl, thiocarbamoyl, fluorine, chlorine, bromine, or represents respectively optionally fluorine- and/or chlorine-substituted alkyl, alkoxy or alkylthio having in each case 1 or 2 carbon atoms,
R³ represents the grouping -A¹-A²-A³
in which
A¹ represents a single bond, represents oxygen or the grouping -N-A⁴- in which A⁴ represents hydrogen, hydroxyl, C₁-C₄-alkyl, C₃-C₄-alkenyl, C₃-C₄-alkinyl, C₁-C₄-alkoxy, phenyl, C₁-C₄-alkylsulphonyl or phenylsulphonyl,
A² represents a single bond or represents optionally fluorine-, chlorine- or bromine-substituted C₁-C₆-alkanediyl,
A³ represents fluorine, chlorine, bromine,
A³ furthermore represents respectively optionally fluorine-, chlorine- or C₁-C₄-alkoxy-substituted alkyl, alkoxy, alkylthio, alkylsulphinyl, alkylsulphonyl, alkylamino, dialkylamino, alkoxycarbonyl or dialkoxy(thio)phosphoryl having in each case 1 to 6 carbon atoms in the alkyl groups,
A³ furthermore represents respectively optionally fluorine- or chlorine-substituted alkenyl, alkenyloxy, alkenylamino, alkylideneamino, alkenyloxycarbonyl, alkinyl, alkinyloxy, alkinylamino or alkinyloxycarbonyl having in each case 2 to 6 carbon atoms in the alkenyl, alkylidene or alkinyl groups, and
R⁴, R⁵ and R⁶ are identical or different and each represents hydrogen, cyano, thiocarbamoyl, nitro, hydroxyl, mercapto, amino, fluorine, chlorine, bromine, or represents respectively optionally fluorine- and/or chlorine-substituted alkyl, alkoxy, alkylthio, alkylamino or dialkylamino having in each case 1 to 4 carbon atoms in the alkyl groups,
except for the compounds 2-(2,4,5-trichloro-phenyl)-pyridazin-3-one and 4-chloro-5-dimethylamino-2-(4-chloro-2-fluoro-5-propargyloxy-phenyl)-pyridazin-3-one.

2. Phenylpyridazinones according to Claim 1, **characterized in that**
R¹ represents fluorine or chlorine,
R² represents cyano, thiocarbamoyl, fluorine, chlorine, bromine, methyl or trifluoromethyl,
R³ represents the grouping -A¹-A²-A³
in which
A¹ represents a single bond, represents oxygen or the grouping -N-A⁴- in which A⁴ represents hydrogen, hydroxyl, methyl, ethyl, n- or i-propyl, methoxy, ethoxy, n- or i-propoxy, methylsulphonyl or ethylsulphonyl,
A² represents a single bond, or represents methylene, ethane-1,1-diyl, ethane-1,2-diyl, propane-1,1-diyl, propane-1,2-diyl or propane-1,3-diyl,
A³ represents fluorine, chlorine, bromine,
A³ furthermore represents respectively optionally fluorine-, chlorine-, methoxy- or ethoxy-substituted methyl, ethyl, n- or i- propyl, n-, i-, s- or t-butyl, n-, i-, s- or t-pentyl, methoxy, ethoxy, n- or i-propoxy, n-, i-, s- or t-butoxy, n-, i-, s- or t-pentyloxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, dimethylamino, diethylamino, methoxycarbonyl, ethoxycarbonyl, n- or i-propoxycarbonyl, dimethoxyphosphoryl, diethoxyphosphoryl, dipropoxyphosphoryl or diisopropoxyphosphoryl, and
A³ furthermore represents respectively optionally fluorine- or chlorine-substituted propenyl, butenyl, propenyloxy, butenyloxy, propenylamino, butenylamino, propylideneamino, butylideneamino, propenyloxycarbonyl, butenyloxycarbonyl, propinyl, butinyl, propinyloxy, butinyloxy, propinylamino, butinylamino, propinyloxycarbonyl or butinyloxycarbonyl, and
R⁴, R⁵ and R⁶ are identical or different and each represents hydrogen, cyano, thiocarbamoyl, nitro, hydroxyl, mercapto, amino, fluorine, chlorine, bromine, or represents respectively optionally fluorine- and/or chlorine-substituted methyl, ethyl, n- or i-propyl, n-, i-, s- or t-butyl, methoxy, ethoxy, methylthio, ethylthio, methylamino, ethylamino or dimethylamino.

3. Process for preparing the phenylpyridazinones according to Claim 1 or 2,
**characterized in that**
(a) halogenoarenes of the general formula (II) 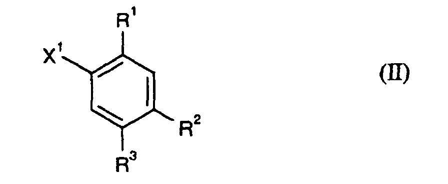 in which
R¹, R² and R³ are each as defined in Claim 1 or 2 and
X¹ represents halogen
are reacted with pyridazinones of the general formula (III) 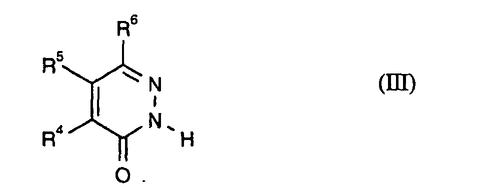 in which
R⁴, R⁵ and R⁶ are each as defined in Claim 1 or 2
- or with acid adducts or alkali metal salts of compounds of the formula (III) -
if appropriate in the presence of a reaction auxiliary and if appropriate in the presence of a diluent,
or
(b) arylhydrazines of the general formula (IV) 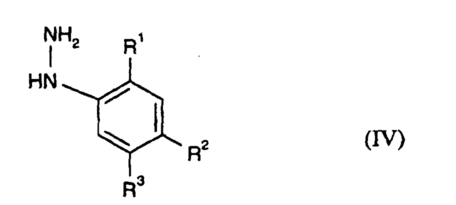 in which
R¹, R² and R³ are each as defined above
are reacted with β-trihalomethyl-enones of the general formula (V) 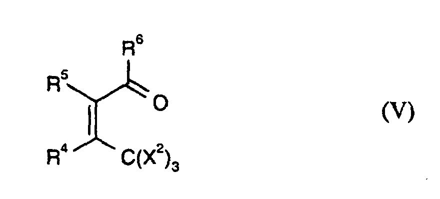 in which
R⁴, R⁵ and R⁶ are each as defined above and
X² represents halogen,
if appropriate in the presence of a reaction auxiliary and if appropriate in the presence of a diluent,
or
(c) hydrazonecarboxylic acids of the general formula (VI) 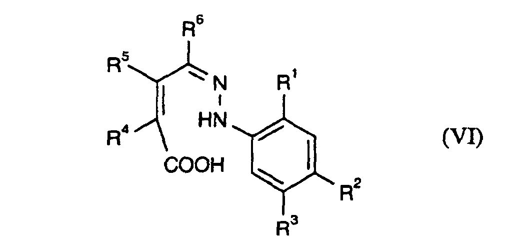 in which
R¹, R², R³, R⁴, R⁵ and R⁶ are each as defined above
are cyclocondensed, i.e. reacted with a dehydrating agent,
or
(d) 2,4-disubstituted phenylpyridazinones of the general formula (Ia) 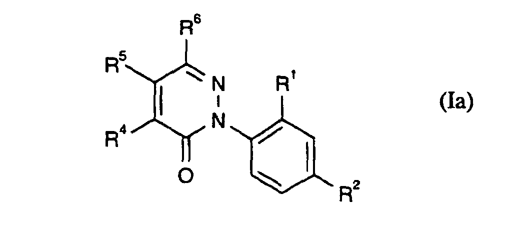 in which
R¹, R², R⁴, R⁵ and R⁶ are each as defined above
are nitrated, i.e. reacted with a nitrating agent.

4. Herbicides, **characterized in that** they comprise at least one phenylpyridazinone according to Claim 1 or 2.

5. Method for controlling undesirable plants, **characterized in that** phenylpyridazinones according to Claim 1 or 2 are allowed to act on undesirable plants and/or their habitat.

6. Use of phenylpyridazinones according to Claim 1 or 2 for controlling undesirable plants.

7. Process for preparing herbicides, **characterized in that** phenylpyridazinones according to Claim 1 or 2 are mixed with extenders and/or surfactants.

8. Hydrazonecarboxylic acids of the general formula (VI) 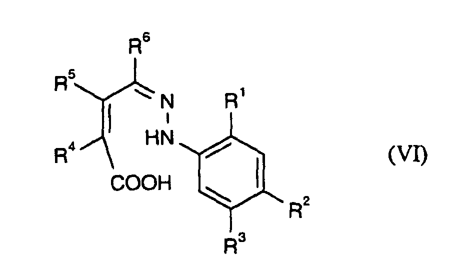 in which
R¹, R², R³, R⁴, R⁵ and R⁶ are each as defined in Claim 1 or 2.

## Revendications

1. Phénylpyridazinones de formule générale (I) 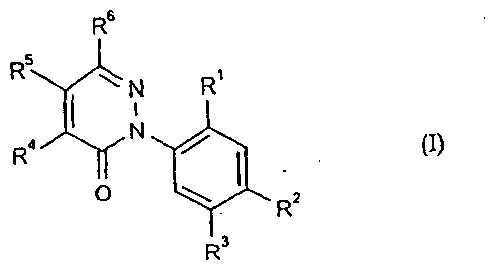 dans laquelle
R¹ représente le fluor, le chlore ou le brome,
R² est un groupe cyano, carbamoyle, thiocarbamoyle, le fluor, le chlore, le brome ou un reste alkyle, alkoxy ou alkylthio ayant chacun 1 ou 2 atomes de carbone et chacun étant éventuellement substitué par du fluor et/ou du chlore,
R³ représente le groupement -A¹-A²-A³,
dans lequel
A¹ est une liaison simple, l'oxygène ou le groupement -N-A⁴-, dans lequel A⁴ est l'hydrogène, le groupe hydroxy, un reste alkyle en C₁ à C₄, alcényle en C₃ ou C₄, alcynyle en C₃ ou C₄, alkoxy en C₁ à C₄, phényle, alkylsulfonyle en C₁ à C₄ ou phénylsulfonyle,
A² est une liaison simple ou un reste alcanediyle en C₁ à C₆ éventuellement substitué par du fluor, du chlore ou du brome,
A³ représente le fluor, le chlore ou le brome,
A³ représente en outre un reste alkyle, alkoxy, alkylthio, alkylsulfinyle, alkylsulfonyle, alkylamino, dialkylamino, alkoxycarbonyle ou dialkoxy(thio)phosphoryle ayant chacun 1 à 6 atomes de carbone dans les groupes alkyles et chacun étant éventuellement substitué par du fluor, du chlore ou un reste alkoxy en Ci à C₄,
A³ représente en outre un reste alcényle, alcényloxy, alcénylamino, alkylidène-amino, alcényloxycarbonyle, alcynyle, alcynyloxy, alcynylamino, alcynyloxy-carbonyle ayant chacun 2 à 6 atomes de carbone dans les groupes alcényle, alkylidène ou alcynyle et chacun étant éventuellement substitué par du fluor ou du chlore, et
R⁴, R⁵ et R⁶ sont identiques ou différents et représentent l'hydrogène, un groupe cyano, thiocarbamoyle, nitro, hydroxy, mercapto, amino, le fluor, le chlore, le brome ou un reste alkyle, alkoxy, alkylthio, alkylamino ou dialkylamino ayant chacun 1 à 4 atomes de carbone dans les groupes alkyles et chacun étant éventuellement substitué par du fluor et/ou du chlore,
à l'exception des composés 2-(2,4,5-trichlorophényl)-pyridazine-3-one et 4-chloro-5-diméthylamino-2-(4-chloro-2-fluoro-5-propargyloxy-phényl)-pyridazine-3-one.

2. Phénylpyridazinones suivant la revendication 1, **caractérisées en ce que**
R¹ représente le fluor ou le chlore,
R² est un groupe cyano, thiocarbamoyle, le fluor, le chlore, le brome, un reste méthyle ou trifluorométhyle,
R³ représente le groupement -A¹-A²-A³,
dans lequel
A¹ représente une liaison simple, l'oxygène ou le groupement -N-A⁴-, dans lequel A⁴ est l'hydrogène, un groupe hydroxy, un reste méthyle, éthyle, n-propyle, isopropyle, méthoxy, éthoxy, n-propoxy, isopropoxy, méthylsulfonyle ou éthylsulfonyle,
A² est une liaison simple ou un reste méthylène, éthane-1,1-diyle, éthane-1,2-diyle, propane-1,1-diyle, propane-1,2-diyle ou propane-1,3-diyle,
A³ représente le fluor, le chlore, le brome,
A³ représente en outre un reste méthyle, éthyle, n-propyle, isopropyle, n-butyle, isobutyle, sec.-butyle, tertiobutyle, n-pentyle, isopentyle, sec.-pentyle, tertiopentyle, méthoxy, éthoxy, n-propoxy, isopropoxy, n-butoxy, isobutoxy, sec.-butoxy, tertiobutoxy, n-pentyloxy, isopentyloxy, sec.-pentyloxy, tertiopentyloxy, méthylthio, éthylthio, n-propylthio, isopropylthio, n-butylthio, isobutylthio, sec.-butylthio, tertiobutylthio, méthylsulfinyle, éthylsulfinyle, n-propyl-sulfinyle, isopropylsulfinyle, méthylsulfonyle, éthylsulfonyle, n-propylsulfonyle, isopropyl-sulfonyle, méthylamino, éthylamino, n-propylamino, isopropylamino, n-butylamino, isobutylamino, sec.-butylamino, tertiobutylamino, diméthylamino, diéthylamino, méthoxycarbonyle, éthoxycarbonyle, n-propoxycarbonyle, isopropoxycarbonyle, diméthoxyphosphoryle, diéthoxyphosporyle, dipropoxyphosphoryle ou diisopropoxyphosphoryle dont chacun porte éventuellement un substituant fluor, chlore, méthoxy ou éthoxy, et
A3 représente en outre un reste propényle, butényle, propényloxy, butényloxy, propénylamino, buténylamino, propylidène-amino, butylidène-amino, propényloxycarbonyle, butényloxycarbonyle, propynyle, butynyle, propynyloxy, butynyloxy, propynylamino, butynylamino, propynyloxycarbonyle ou butynyloxycarbonyle dont chacun est éventuellement substitué par du fluor ou du chlore,
et
R⁴, R⁵ et R⁶ sont identiques ou différents et représentent l'hydrogène, un groupe cyano, thiocarbamoyle, nitro, hydroxy, mercapto, amino, le fluor, le chlore, le brome ou un reste méthyle, éthyle, n-propyle, isopropyle, n-butyle, isobutyle, sec.-butyle, tertiobutyle, méthoxy, éthoxy, méthylthio, éthylthio, méthylamino, éthylamino ou diméthylamino dont chacun est éventuellement substitué par du fluor et/ou du chlore.

3. Procédé de production des phénylpyridazinones suivant la revendication 1 ou 2, **caractérisé en ce que** :
(a) on fait réagir des halogénarènes de formule générale (II) 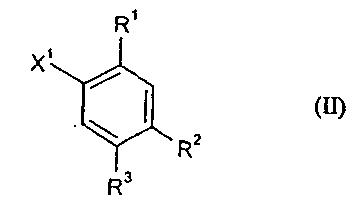 dans laquelle
R¹, R² et R³ ont les définitions indiquées dans la revendication 1 ou 2 et
X¹ est un halogène,
avec des pyridazinones de formule générale (III) 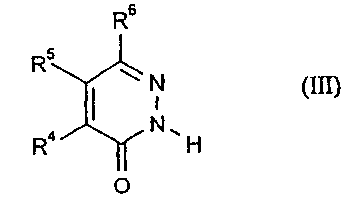 dans laquelle
R⁴, R⁵ et R⁶ ont les définitions indiquées dans la revendication 1 ou 2,
ou avec des produits d'addition d'acides ou des sels de métaux alcalins de.composés de formule (III) -
éventuellement en présence d'un auxiliaire de réaction et, le cas échéant, en présence d'un diluant,
ou **en ce que** :
(b) on fait réagir des arylhydrazines de formule générale (IV) 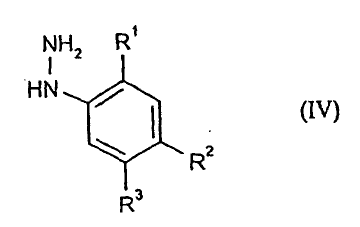 dans laquelle
R¹, R² et R³ ont les définitions indiquées ci-dessus,
avec des β-trihalogénométhyl-énones de formule générale (V) 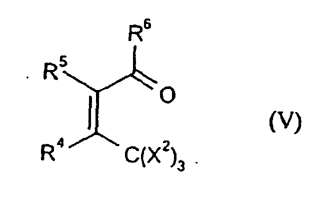 dans laquelle
R⁴, R⁵ et R⁶ ont les définitions indiquées ci-dessus et
X² est un halogène,
éventuellement en présence d'un auxiliaire de réaction et, le cas échéant, en présence d'un diluant,
ou **en ce que** :
(c) on cyclise par condensation, c'est-à-dire par réaction avec un agent déshydratant, des acides hydrazonecarboxyliques de formule générale (VI) 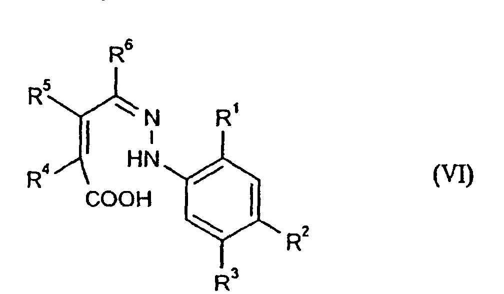 dans laquelle R³
R¹, R², R³, R⁴, R⁵ et R⁶ ont les définitions indiquées ci-dessus
ou **en ce que**
(d) on nitre, c'est-à-dire qu'on fait réagir avec un agent de nitration, des phénylpyridazinones disubstituées en positions 2 et 4, de formule générale (Ia) 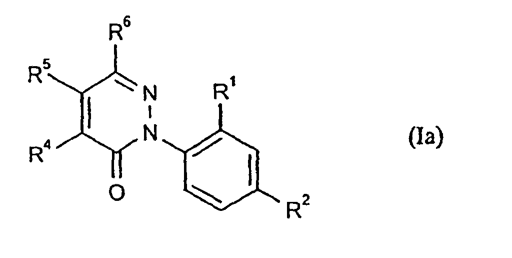
dans laquelle
R¹, R², R⁴, R⁵ et R⁶ ont les définitions indiquées ci-dessus

4. Composition d'herbicide, **caractérisée par** une teneur en au moins une phénylpyridazinone suivant la revendication 1 ou 2.

5. Procédé pour combattre des plantes indésirables, **caractérisé en ce qu'**on fait agir des phénylpyridazinones suivant la revendication 1 ou 2 sur les plantes indésirables et/ou sur leur milieu.

6. Utilisation de phénylpyridazinones suivant la revendication 1 ou 2 pour combattre des plantes indésirables.

7. Procédé de préparation de compositions herbicides, **caractérisé en ce qu'**on mélange des phénylpyridazinones suivant la revendication 1 ou 2 avec des diluants et/ou des substances tensioactives.

8. Acides hydrazonecarboxyliques de formule générale (VI) 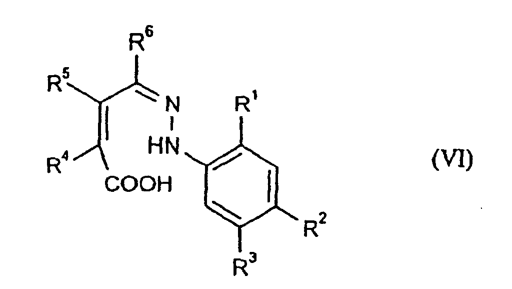 dans laquelle
R¹, R², R³, R⁴, R⁵ et R⁶ ont les définitions indiquées dans la revendication 1 ou 2.
